# EUROPÄISCHE PATENTSCHRIFT

(11) **EP 0 630 880 B1**
(45) Veröffentlichungstag und Bekanntmachung des Hinweises auf die Patenterteilung: **12.03.1997**
(21) Anmeldenummer: 94109133.2
(22) Anmeldetag: 15.06.1994
(51) Int. Cl.: C07C 67/14, C07C 69/80, C07C 67/60

(54) **Verfahren zur Herstellung von Carbonsäureestern**
Method of preparing carboxylic acid esters
Procédé pour la préparation d'esters d'acides carboxyliques

(30) Priorität: 23.06.1993 DE 4320820
(43) Veröffentlichungstag der Anmeldung: 28.12.1994
(73) Patentinhaber: BASF Aktiengesellschaft, 67063 Ludwigshafen (DE)
(72) Erfinder: Therre, Joerg, Dr., D-67550 Worms (DE); Schwarz, Hans Volkmar, Dr., B-1410 Waterloo (BE); Agar, David, Dr., D-68161 Mannheim (DE)

(56) Entgegenhaltungen:
- DE-B- 1 292 655
- JOURNAL OF PHARMACEUTICAL SCIENCES, Bd.56, Nr.11, 1967, Washington, US, Seiten 1446-1453, J. NEMATOLLAHI et al: "Plasticizers in medical applications I"

## Beschreibung

Die Erfindung betrifft ein Verfahren zur Umsetzung von in Carbonsäureestern gelösten Carbonsäurechloriden insbesondere wendbar zum Aufarbeiten von Carbonsäurediestern, die als sungsmittel bei der Herstellung von Isocyanaten durch Phosgenierung von Aminen verwendet werden.

Die Herstellung von Isocyanaten, insbesondere von Di- oder Polyisocyanaten durch Umsetzung der entsprechenden Amine mit Phosgen ist allgemein bekannt und vielfach in der Literatur vorbeschrieben. Zur Durchführung des Verfahrens werden zumeist sowohl das Amin als auch das Phosgen in einem Lösungsmittel gelöst und zur Reaktion gebracht. Als Lösungsmittel können beispielsweise insbesondere aromatische, gegebenenfalls halogenierte Kohlenwasserstoffe wie Toluol oder Chlorbenzole zum Einsatz kommen. Möglich ist auch der Einsatz von Carbonsäurealkylestern als Lösungsmittel bei der Phosgenierung von Aminen. Hierbei ist aromatischen Estern der Vorzug zu geben, da diese sich bei den angewendeten Reaktionsbedingungen chemisch weitgehend inert verhalten.

So beschreibt die FR-A 1 476 755 die Verwendung von Alkylisophthalaten als Lösungsmittel bei der Umsetzung von Toluylendiamin mit Phosgen zu Toluylendiisocyanat. Während der nierungsreaktion entstehen durch die Einwirkung von Phosgen und/oder Chlorwasserstoff auf das Lösungsmittel Carbonsäurealkylesterchloride sowie in untergeordnetem Maße Carbonsäuredichloride. Damit geht Lösungsmittel verloren, was zu einer Kostensteigerung des Verfahrens führt.

Da diese Umsetzungsprodukte bei der destillativen Aufarbeitung des Reaktionsgemisches im Lösungsmittel verbleiben, werden sie mit dem Lösungsmittel mitgeführt und reichern sich darin an. Sie können bis zu 40 % des Lösungsmittels ausmachen. Dabei reagieren die Säurechloride mit dem Ausgangsprodukt Toluylendiamin, was zu einer Ausbeutesenkung des Verfahrens führt. Diese Reaktionsprodukte können sich in der Anlage ablagern und so zu Störungen in der Anlage führen. Da eine destillative Entfernung der Säurechloride nicht möglich ist, müssen sie auf andere geeignete Weise aus dem Reaktionsgemisch entfernt werden, am besten durch Rückreaktion zum entsprechenden Ester.

Zur Umwandlung von Carbonsäurechloriden in Carbonsäureester gibt es im Stand der Technik eine Reihe von Vorschlägen. Im Z. org. Chim 1 (1965) Nr. 8, S. 1396-1399, wird vorgeschlagen, Terephthalsäurealkylesterchlorid mit Quecksilberdiacetaldehyd in Gegenwart von Pyridin zu Terephthalsäurevinylalkylester umzuwandeln. Der Nachteil dieses Verfahrens liegt in der Notwendigkeit der Verwendung von Quecksilberderivaten sowie des Hilfsstoffes Pyridin.

Allgemein bekannt ist die Möglichkeit, Säurechloride mit Alkoholen zu Estern umzusetzen. Dabei wird allerdings im Stand der Technik die Meinung vertreten, daß für die Reaktion von aromatischen Carbonsäurechloriden mit Alkoholen Hilfsstoffe und/oder Lösungsmittel erforderlich seien, sowie der entstehende Chlorwasserstoff durch die Zugabe von Chlorwasserstoff bindenden Mitteln wie Basen, insbesondere Pyridin, oder durch Durchleiten von Stickstoff bzw. Anlegen von Vakuum aus dem Reaktionsgemisch entfernt werden müssen. Dadurch sind die bekannten Verfahren sehr aufwendig, haben lange Reaktionszeiten, schlechte Ausbeuten und sind daher zur Reinigung der als Lösungsmittel für die Isocyanatherstellung eingesetzten Carbonsäureester nicht geeignet.

So werden in Houben-Weyl, vierte Auflage (1952), Band VIII, Seite 545-547, zahlreiche Möglichkeiten beschrieben, die Reaktion des Säurechlorids mit dem Alkohol bei Anwesenheit von Chlorwasserstoff-bindenden Hilfsstoffen wie Basen oder Pyridin durchzuführen. Dies bedeutet jedoch in der Regel den Verlust des Chlorwasserstoff bindenden Hilfsstoffes sowie die Notwendigkeit, die so entstehenden Reaktionsprodukte aus dem tionsgemisch zu entfernen.

In DE-A 12 92 655 wird vorgeschlagen, aufgrund der Reaktionsträgheit der aromatischen Säurechloride den entstehenden wasserstoff durch säurebindende Mittel aus dem Reaktionsgleichgewicht zu entfernen oder die Reaktion in einem polaren genkohlenwasserstoff als Lösungsmittel durchzuführen. Dieses Lösungsmittel muß nach der Reaktion durch Destillation entfernt werden.

Im Journal of Pharmaceutical Sciences, Vol. 56, Nr. 11 (1967), S. 1448, wird in einer allgemeinen Arbeitsvorschrift empfohlen, ein aromatisches Carbonsäuredichlorid mit einem Alkohol 4 bis 6 Stunden unter Rückfluß zu kochen. Die Nachteile des Verfahrens liegen in einer sehr langen Reaktionszeit, der geringen Ausbeute von lediglich 55 bis 75 % sowie der Notwendigkeit, das Reaktionsprodukt aufwendig zu reinigen.

Im Journal für praktische Chemie, 4. Reihe, Band 27, S. 236-238, wird die Umsetzung von Isophthalsäuredichlorid mit Isopropanol, Isobutanol und Isopentanol beschrieben. Die Nachteile des Verfahrens liegen in einem sehr hohen sechsfachen Überschuß des Alkohols, einer Reaktionszeit von 10 Stunden und einer Ausbeute von weniger als 37 %.

In US-A 4 379 912 werden Carbonsäuredichloride bei Temperaturen von 180 bis 220°C in Gegenwart von Zinkacetat und Antimonoxid zu Estern umgesetzt. Nachteilig ist hierbei, daß die Katalysatoren in einem speziellen Reinigungsschritt abgetrennt werden müssen.

In US-A 2 623 034 wird zur Herstellung von kristallinen Tetramethylen-Isophthalatpolymeren durch die Umsetzung von phthalsäuredichlorid mit Tetramethylenglykol empfohlen, den entstehenden Chlorwasserstoff durch Spülen mit Stickstoff oder das Anlegen von Vakuum zu entfernen. Dieses Verfahren kann nicht angewendet werden, wenn leichtflüchtige niedere Alkanole als Alkohol eingesetzt werden.

Die im Stand der Technik beschriebenen Verfahren sind also mit erheblichen Nachteilen wie schlechte Ausbeute, langsame Reaktion sowie der Benutzung von Katalysatoren Hilfsstoffen oder Lösungsmitteln, behaftet. Für die Aufarbeitung von als nierungslösungsmitteln eingesetzten Carbonsäurediestern sind sie nicht geeignet.

Die Aufgabe der Erfindung bestand darin, ein Verfahren zur Umwandlung von in Carbonsäuredialkylestern, insbesondere solche auf der Basis aromatischer Carbonsäuren, gelösten Carbonsäurechloriden in die entsprechenden Carbonsäuredialkylsäureester zu entwickeln, das die Nachteile des Standes der Technik vermeidet. Die Aufgabe konnte überraschenderweise gelöst werden durch ein Verfahren zur Umsetzung von in Carbonsäuredialkylestern gelösten entsprechenden Carbonsäurealkylesterchloriden und/oder Carbonsäuredichloriden mit dem entsprechenden Alkanol bei Temperaturen von 60 bis 300°C, insbesondere 100 bis 250°C, wobei der entstehende Chlorwasserstoff im Reaktionsgemisch verbleibt.

Gegenstand der Erfindung ist demzufolge ein Verfahren zur Umsetzung von in Carbonsäuredialkylestern gelösten entsprechenden Carbonsäurealkylesterchloriden und/oder Carbonsäuredichloriden mit dem entsprechenden Alkanol bei Temperaturen von 60 bis 300°C, insbesondere von 100 bis 250°C, wobei der entstehende Chlorwasserstoff im Reaktionsgemisch verbleibt.

Die Reaktionszeiten betragen dabei, insbesondere in Abhängigkeit von der Konzentration der Carbonsäurechloride im Dialkylester, 1 Sekunde bis 4 Stunden, insbesondere 1 Minute bis 3 Stunden. Die Umsetzung wird vorgenommen, indem man den Carbonsäuredialkylester, der zwischen 0,05 bis 40 Gew.-%, bezogen auf das Gesamtgewicht, an Säurechloriden aufweist, mit dem dem Carbonsäurendialkylester entsprechenden Alkohol mischt, das Reaktionsgemisch auf die angegebene Reaktionstemperatur bringt, die Reaktionsmischung bis zur vollständigen Umsetzung der Säurechloride bei dieser Temperatur beläßt und danach abkühlen läßt.

Die Reaktionszeit beträgt, abhängig von der Menge der Säurechloride, 1 Sekunde bis 4 Stunden. Sie läßt sich durch Vorversuche leicht ermitteln.

Der sich bei der Reaktion bildende Chlorwasserstoff verbleibt während der gesamten Reaktionszeit im Reaktionsgemisch. Wenn ein vollständiger Umsatz angestrebt wird, ist es notwendig, daß das Molverhältnis von Säurechloridgruppen und Alkohol mindestens 1:1 beträgt. Zur Beschleunigung ist es vorteilhaft, mit einem Alkoholüberschuß zu arbeiten. Dieser Überschuß sollte 0,005 mol/kg bis 10 mol/kg, vorzugsweise 0,05 bis 5 mol/kg betragen.

Der nicht umgesetzte Alkohol kann nach beendeter Umsetzung auf an sich bekannte Weise aus dem Reaktionsgemisch entfernt werden, z.B. durch Destillation oder Durchleiten von Inertgas. Ein höherer als der angegebene Alkoholüberschuß ist dabei für die Reaktion nicht nachteilig. Durch den erforderlichen Aufwand zur Entfernung des nicht umgesetzten Alkohols steigt allerdings der Aufwand des Verfahrens.

Gemeinsam mit dem nicht umgesetzten Alkohol wird auch der bei der Reaktion gebildete Chlorwasserstoff aus dem Reaktionsgemisch entfernt. Der Alkohol kann vor Beginn der Reaktion in Carbonsäureester vorgelöst und dann mit dem Säurechlorid enthaltenden Carbonsäureester vereinigt werden. Es ist aber auch möglich, den Alkohol als Reinstoff zu verwenden.

Das Verfahren erlaubt es, die Umsetzung zwischen dem Säurechlorid und dem Alkohol innerhalb kurzer Zeit, oft sogar innerhalb weniger Minuten zu beenden. Das bedeutet, daß nur kleine Apparate benötigt werden. Die Reaktion kann sowohl diskontinuierlich als auch kontinuierlich durchgeführt werden. Als Apparate eignen sich sowohl Rohrreaktoren als auch Rührkessel. Der Druck im Reaktionsgefäß hat auf die Reaktion keinen Einfluß. Es kann mit dem Druck gearbeitet werden, der sich bei der Reaktionstemperatur im Reaktionsgefäß einstellt. In der Regel liegt dieser Druck unterhalb von 50 bar, oft auch unterhalb von 17 bar.

Abgesehen von der genannten eventuellen Abtrennung von überschüssigem Alkohol ist es nicht notwendig, das Reaktionsprodukt einer Reinigung zuzuführen. Besonders vorteilhaft kann das erfindungsgemäße Verfahren bei der Reinigung von als Lösungsmittel bei der Phosgenierung von Toluylendiamin zu Toluylendiisocyanat verwendeten Dialkylisophthalaten eingesetzt werden. Als Dialkylisophthalate können hierbei alle Produkte mit Alkanolen von 1 bis 20 Kohlenstoffatomen in der Hauptkette insbesondere Methyl-, Ethyl-, Propyl-, Butyl-, Octyl-, insbesondere Ethylgruppen eingesetzt werden.

Unter der Einwirkung von Phosgen und/oder Chlorwasserstoff wandelt sich bei der Phosgenierung von Toluylendiamin zu Toluylendiisocyanat das Dialkylisophthalat teilweise in phthalsäurechlorid um. Das erfindungsgemäße Verfahren gestattet es, nach der Phosgenierung des Toluylendiamins, der Abtrennung des überschüssigen Phosgens und der leicht flüchtigen Bestandteile sowie des Toluylendiisocyanates das mit Säurechlorid verunreinigte Phosgenierungslösungsmittel aufzuarbeiten. Dazu wird das Lösungsmittel mit dem entsprechenden Alkohol, dessen Menge sich nach der Verunreinigung des Lösungsmittels mit Säurechlorid richtet, versetzt und auf die Reaktionstemperatur erwärmt.

Wird mit einer überstöchiometrischen Menge Alkohol gearbeitet, ist es notwendig, das Lösungsmittel destillativ zur Abtrennung des überschüssigen Alkohols zu behandeln. Bei der Alkoholabtrennung wird gleichzeitig auch der Chlorwasserstoff aus dem Reaktionsgemisch entfernt. Wird mit einer unterstöchiometrischen oder stöchiometrischen Alkoholmenge gearbeitet, ist eine destillative Aufarbeitung des Reaktionsproduktes nicht erforderlich, das Lösungsmittel kann mit dem darin enthaltenen Chlorwasserstoff wieder zur Phosgenierung von TDA zu TDI verwendet werden.

Die Aufarbeitung des in der Technik bevorzugt als Phosgenierungslösungsmittel verwendeten Diethylisophthalats erfolgt vorzugsweise bei 160 bis 240°C.

Die Erfindung soll an nachstehenden Beispielen näher erläutert werden.

### Beispiele

### Beispiel 1

Es wurden zwei Lösungen hergestellt. Lösung 1 bestand aus 67,5001 g Diethylisophthalat, 15,0031 g Isophthalsäuredichlorid und als internen Standard 0,4005 g Eicosan. Die zweite Lösung bestand aus 67,5001 g Diethylisophthalat und 16,9993 g Ethanol. Die beiden Lösungen wurden in einem 300 ml Autoklaven vereinigt, 35 bar Stickstoff aufgepreßt und die Heizung eingeschaltet. Nach 25 min waren 120°C erreicht. 10 min nach Erreichen dieser Temperatur wurde eine Probe aus dem Autoklaven entnommen, in der durch Gaschromatographie kein Isophthalsäuredichlorid mehr nachweisbar war. Die Konzentration an Isophthalsäureethylesterchlorid betrug laut Gaschromatographie 0,00036 mol/kg. 30 min nach Erreichen der Versuchstemperatur wurde eine weitere Probe aus dem Autoklaven entnommen, in der durch Gaschromatographie kein Isophthalsäureethylesterchlorid mehr nachweisbar war.

### Beispiel 2 (Herstellung einer Lösung von Isophthalsäureethylesterchlorid in Diethylisophthalat)

450,0 g Diethylisophthalat, 100,05 g Isophthalsäuredichlorid und 67,90 g entwässertes Ethanol wurden gemischt und 60 min lang bei 50°C gerührt. Dann wurde bei 20°C der entstandene Chlorwasserstoff und das nicht umgesetzte Ethanol durch Anlegen von Vakuum abdestilliert. Es wurden 555,52 g einer Lösung, die laut gaschromatographischer Analyse 0,47 mol/kg Isophthalsäureethylesterchlorid enthielt, erhalten.

### Beispiel 3

Von der nach Beispiel 2 hergestellten Lösung sowie von einer Lösung von 14,57 g wasserfreiem Ethanol in 539,95 g Diethylisophthalat wurden mittels zweier Pumpen pro Zeiteinheit gleiche Volumina gefördert, auf 160°C erhitzt, dann gemischt und durch einen auf 160°C erhitzten Rohrreaktor mit einem Volumen von 42,4 ml gepumpt, in dem die Mischung der Lösungen 4 min verweilte. Durch ein Ventil wurde der Druck im Reaktor auf 21 bar eingestellt. Die aus dem Rohrreaktor austretende Lösung wurde mittels Gaschromatographie analysiert. Die Analyse ergab einen Gehalt an Isophthalsäureethylesterchlorid von nur noch 0,079 mol/kg.

### Beispiel 4

Es wurde wie in Beispiel 3 verfahren, die Temperatur betrug jedoch 200°C. Die aus dem Rohrreaktor austretende Lösung wurde mittels Gaschromatographie analysiert. Die Analyse ergab einen Gehalt an Isophthalsäureethylesterchlorid von nur noch 0,008 mol/kg.

### Beispiel 5

Von einer nach Beispiel 2 hergestellten Lösung, die laut gaschromatographischer Analyse 0,49 mol/kg Isophthalsäureethylesterchlorid enthielt, sowie von einer Lösung von 8,16 g wasserfreiem Ethanol und 191,85 g Diethylisophthalat wurden mittels zweier Pumpen pro Zeiteinheit gleiche Volumina gefördert, auf 197°C erhitzt, gemischt und durch einen auf 197°C erhitzten Rohrreaktor mit einem Volumen von 42,4 ml gepumpt, in dem die Mischung der Lösung 30 min verweilte. Durch ein Ventil war der Druck in dem Reaktor auf 17 bar eingestellt. Die aus dem Rohrreaktor austretende Lösung wurde mittels Gaschromatographie analysiert. Es wurde kein Isophthalsäureesterchlorid mehr nachgewiesen.

## Patentansprüche

1. Verfahren zur Umsetzung von in Carbonsäuredialkylestern gelösten entsprechenden Carbonsäurealkylesterchloriden und/oder Carbonsäuredichloriden zu Carbonsäuredialkylestern, dadurch gekennzeichnet, daß dieses Gemisch mit dem entsprechenden Alkanol bei Temperaturen von 60 bis 300°C umgesetzt wird, wobei der entstehende Chlorwasserstoff im Reaktionsgemisch verbleibt.

2. Verfahren nach Anspruch 1, dadurch gekennzeichnet, daß die Umsetzung bei Temperaturen von 100 bis 250°C verläuft.

3. Verfahren nach Anspruch 1, dadurch gekennzeichnet, daß die Carbonsäuredialkylester insbesondere solche von aromatischen Dicarbonsäuren sind.

4. Verfahren nach Anspruch 1 bis 3, dadurch gekennzeichnet, daß die Carbonsäuredialkylester Isophthalsäuredialkylester sind.

5. Verfahren nach Anspruch 4, dadurch gekennzeichnet, daß die Isophthalsäuredialkylester Isophthalsäurediethylester sind.

6. Verfahren zur Aufarbeitung von als Phosgenierungslösungsmittel bei der Toluylendiisocyanatherstellung verwendeten mit Isophthalsäureethylesterchlorid und Isophthalsäuredichlorid verunreinigtem Isophthalsäurediethylester, dadurch gekennzeichnet, daß dieses Gemisch bei 60 bis 300°C mit Ethanol umgesetzt wird, wobei der entstehende Chlorwasserstoff im Reaktionsgemisch verbleibt.

## Claims

1. A process for reacting alkyl ester chlorides of carboxylic acids and/or carboxylic acid dichlorides dissolved in corresponding dialkyl carboxylates to give dialkyl carboxylates, which comprises reacting this mixture with the corresponding alkanol at from 60 to 300°C, with the hydrogen chloride formed remaining in the reaction mixture.

2. A process as claimed in claim 1, wherein the reaction occurs at from 100 to 250°C.

3. A process as claimed in claim 1, wherein the dialkyl carboxylates are, in particular, those of aromatic dicarboxylic acids.

4. A process as claimed in any of claims 1 to 3, wherein the dialkyl carboxylates are dialkyl isophthalates.

5. A process as claimed in claim 4, wherein the dialkyl isophthalates are diethyl isophthalates.

6. A process for working up diethyl isophthalates used as phosgenation solvent in the preparation of tolylene diisocyanate and contaminated with the ethyl ester chloride of isophthalic acid and isophthalic acid dichloride, which comprises reacting this mixture at from 60 to 300°C with ethanol, with the hydrogen chloride formed remaining in the reaction mixture.

## Revendications

1. Procédé de conversion de dichlorures d'acides carboxyliques et/ou de chlorures d'esters alkyliques d'acides carboxyliques correspondants dissous dans des esters dialkyliques d'acides carboxyliques en esters dialkyliques d'acides carboxyliques, caractérisé en ce que l'on fait réagir ce mélange avec l'alcanol correspondant, à des températures de 60 à 300°C, où l'acide chlorhydrique qui se forme demeure dans le mélange réactionnel.

2. Procédé suivant la revendication 1, caractérisé en ce que la réaction se déroule à des températures de 100 à 250°C.

3. Procédé suivant la revendication 1, caractérisé en ce que les esters dialkyliques d'acides carboxyliques sont, plus particulièrement, ceux d'acides dicarboxyliques aromatiques.

4. Procédé suivant les revendications 1 à 3, caractérisé en ce que les esters dialkyliques d'acides carboxyliques sont des esters dialkyliques de l'acide isophtalique.

5. Procédé suivant la revendication 4, caractérisé en ce que les esters dialkyliques de l'acide isophtalique sont l'ester diéthylique de l'acide isophtalique.

6. Procédé de traitement de l'ester diéthylique de l'acide isophtalique contaminé de dichlorure de l'acide isophtalique et de chlorure de l'ester éthylique de l'acide isophtalique, que l'on utilise, à titre de solvant de phosgénation, au cours de la préparation du diisocyanate de toluylène, caractérisé en ce que l'on fait réagir ce mélange avec de l'éthanol à 60 à 300°C, où l'acide chlorhydrique qui se forme subsiste dans le mélange réactionnel.
